# EUROPEAN PATENT APPLICATION

(11) **EP 4 640 632 A1**
(43) Date of publication of application: **29.10.2025**
(21) Application number: 23906870.3
(22) Date of filing: 13.12.2023
(51) Int. Cl.: C01F 7/02, C01F 7/442, C09C 1/28, C09C 1/40, C09D 5/29, C09D 7/61, C09D 201/00

(54) **HIGH-LUMINANCE SCALY a-ALUMINA POWDER AND METHOD FOR PRODUCING SAME**

(30) Priority: 19.12.2022 JP 2022202325
(71) Applicant: Nippon Light Metal Company, Ltd., Tokyo 105-0004 (JP)
(72) Inventor: HAYASHI Daichi, Shizuoka-shi, Shizuoka 424-0901 (JP); IDA Takeo, Shizuoka-shi, Shizuoka 424-0901 (JP); ITO Hiroki, Shizuoka-shi, Shizuoka 424-0901 (JP)
(74) Representative: Plasseraud IP
(86) International application number: PCT/JP2023/044735
(87) International publication number: WO 2024/135505

(57) **Abstract**

Provided is a thin-layered α-alumina powder having a large average particle diameter and aspect ratio as well as excellent perceived luminosity, and a production method capable of obtaining the thin-layered alumina powder by a simple technique. This high-luminance thin-layered α-alumina powder has an average particle diameter of 20 µm to 200 µm, an average thickness of 0.20 µm to 2.00 µm, and an average aspect ratio of 50 to 600. Also, a perceived luminosity measurement reveals that the luminosity intensity Si at a measurement angle θ of 15° and 45° falls in the range from 1.00 to 15.00.

## Description

### TECHNICAL FIELD

The present invention relates to a high-luminance thin-layered (scaly) α-alumina powder and a method for producing the same.

### BACKGROUND ART

α-alumina (Al₂O₃) is chemically stable, excellent in heat resistance, corrosion resistance, wear resistance, and insulation properties, and has high strength and hardness. By taking advantage of the characteristics, an α-alumina powder is widely used in various applications such as structural members, tools, abrasives, fillers, spark plugs, insulators, electronic substrates, and refractory materials.

In particular, a thin-layered α-alumina powder has a high light reflectance on a particle surface and a high luminance. Therefore, by taking advantage of the characteristics, a thin-layered α-alumina powder is used as a high luminance pigment to be added to a coating or a cosmetic. In addition, in a pigment application, an inorganic coating made of TiO₂ or the like is applied to the particle surface of the thin-layered α-alumina powder. Since the coating causes interference of light, it is considered that glossiness is increased by using the interference.

Further, the thin-layered α-alumina powder has high strength and excellent gas barrier properties. Therefore, the thin-layered α-alumina powder is used as a reinforcing material or a gas barrier material to be added to plastics or resin films. The thin-layered α-alumina powder is a powder composed of thin-layered α-alumina particles, and is also referred to as a flaky alumina powder, a flat alumina powder, a plate-like alumina powder, or an alumina flake.

Patent Document 1 discloses an Al₂O₃ flake having a thickness of 500 nm or more, a D₅₀ value of 15 µm to 30 µm, and a D₉₀ value of 30 µm to 45 µm, and discloses that the Al₂O₃ flake has high chemical stability, a smooth surface, and high whiteness at the same time, and is used as a pigment base material (Claim 1, and paragraphs [0010] and [0015] of Patent Document 1). In addition, Patent Document 1 discloses that the Al₂O₃ flake is coated with a high refractive index layer such as TiO₂ or a low refractive index layer such as SiO₂, and is imparted with thus increased gloss, interference color, or a color flop effect (paragraphs [0040] and [0046] of Patent Document 1).

Patent Document 2 discloses a luster pigment-containing coating composition containing an alumina flake as a luster pigment, and discloses that the coating composition can be suitably used as an automatic vehicle top coating, and that a composite coating film formed has novel strong luminosity and novel high designability without deteriorating a finished appearance or the like (Claim 1, and paragraphs [0001] and [0108] of Patent Document 2). In addition, Patent Document 2 discloses that the alumina flake is a flake in which aluminum oxide (Al₂O₃) is coated with a metal oxide such as titanium dioxide, and has a particle size of 10 µm to 30 µm and a thickness of 0.3 µm to 0.4 µm (paragraph [0007] of Patent Document 2).

Patent Document 3 discloses a pearlescent pigment containing aluminum oxide and zinc oxide as main components in a mass ratio of 100:0.1 to 5 and containing a flaky alumina crystalline coated with a metal or metal precursor particles (Claim 1 of Patent Document 3). In addition, Patent Document 3 discloses that the crystalline has an average particle thickness of 0.5 µm or less, an average particle size of 15 µm or more, and an aspect ratio of 50 or more, and thus has excellent glossiness (paragraph [0001] of Patent Document 3).

Patent Document 4 discloses a method for producing a plate-like alumina-based powder having a crystal structure of α-alumina alone, β-alumina alone, or γ-alumina alone or two or more kinds of crystal structures by calcining plate-like boehmite at 400°C to 1,500°C (Claim 2 of Patent Document 4). In addition, Patent Document 4 discloses that a cosmetic having a good feeling of use can be obtained by coating a surface of the plate-like boehmite or the plate-like alumina powder with a hydrophobic compound such as polysiloxane and blending the coated powder with the cosmetic (paragraph [0013] of Patent Document 4).

Patent Document 5 discloses hexagonal plate-like alumina obtained by calcining hexagonal plate-like boehmite at a temperature of 450°C to 1,500°C, the hexagonal plate-like alumina having a substantially hexagonal plate shape, having a ratio of a major axis diameter to a minor axis diameter of 1 to 1.3, and having an aspect ratio of 40 to 100. Patent Document 5 discloses that the hexagonal plate-like alumina has high orientation, a high turbulent reflection and further increased luminosity, and therefore, can be suitably used as a filler for the purpose of luminosity of a coating or a cosmetic (Claim 4 and paragraph [0045] of Patent Document 5).

Patent Document 6 discloses a plate-like alumina particle which has a thickness of 0.01 µm to 5 µm, an average particle diameter of 0.1 µm to 500 µm, an aspect ratio, i.e., a ratio of the particle diameter to the thickness, of 2 to 500, has a polygonal plate shape, and contains molybdenum in the particle (Claim 1 of Patent Document 6). Patent Document 6 discloses that the plate-like alumina particle can be suitably used for a thermally conductive filler, a cosmetic, an abrasive, a highly bright pigment, a lubricant, a base body of a conductive powder, a ceramic material, and the like (paragraph [0101] of Patent Document 6).

### Citation List

### Patent Document

Patent Document 1: Japanese Unexamined Patent Application, Publication No. 2014-218424
Patent Document 2: Japanese Unexamined Patent Application, Publication No. H10-298458
Patent Document 3: Japanese Unexamined Patent Application (Translation of PCT Application), Publication No. 2010-502774
Patent Document 4: Japanese Unexamined Patent Application, Publication No. 2012-071996
Patent Document 5: Japanese Unexamined Patent Application, Publication No. 2003-002642
Patent Document 6: Japanese Unexamined Patent Application, Publication No. 2019-123664

### DISCLOSURE OF THE INVENTION

### Problems to be Solved by the Invention

As described above, in the fields of coatings, cosmetics, and the like, it has been proposed to use a thin-layered α-alumina powder as a high-luminance pigment in the related art. However, the present inventors have found that there is room for improvement in the related art. That is, it is desirable to enhance the luminosity of the alumina powder when used as a high-luminance pigment. However, a thin-layered α-alumina powder in the related art has a limit in increasing reflectance at the particle surface.

In particular, with regard to a thin-layered alumina powder in the related art, it was difficult to increase an average particle diameter and an aspect ratio thereof. That is, as the average particle diameter and the aspect ratio are increased, an area of a plate surface of flat particles is increased. Therefore, even when a surface reflectance is the same, a larger area of the plate surface makes it possible to obtain an alumina powder having more excellent luminosity. In the alumina powder in the related art, the average particle diameter and the aspect ratio were not sufficiently large, and there was a limit in enhancing the luminosity.

Further, a technique for improving glossiness by utilizing an interference effect of light by the inorganic coating is proposed, e.g., Patent Documents 1 to 3, but a thin-layered α-alumina powder cannot be obtained at a low cost with such a technique. That is, after the thin-layered α-alumina powder is prepared, it is necessary to separately form an inorganic coating such as TiO₂ on the surface thereof, which may increase production costs.

The present inventors conducted intensive studies in view of such problems in the related art. As a result, the present inventors have found that the thin-layered α-alumina powder can be obtained by a simple method of calcining a raw material mixture containing a thin-layered boehmite powder and an additive having a predetermined composition. The Present inventors also have found that the obtained thin-layered α-alumina powder has a large average particle diameter and a large aspect ratio and has excellent luminosity.

The present invention has been accomplished on the basis of such findings. An object of the present invention is to provide a thin-layered α-alumina powder having a large average particle diameter and a large aspect ratio and having excellent luminosity, and a method for producing the thin-layered α-alumina powder by a simple method.

### Means for Solving the Problems

The present invention includes the following aspects (1) to (13). In the present description, the expression "to" includes numerical values at both ends thereof. That is, "X to Y" has the same meaning as "X or more and Y or less". In addition, in the present description, any combination of preferred embodiments can be adopted as long as technical harmonization can be achieved. For example, one and the other of preferable numerical ranges can be optionally combined.

(1) A high-luminance thin-layered α-alumina powder having an average particle diameter of 20 µm or more and 200 µm or less, an average thickness of 0.20 µm or more and 2.00 µm or less, and an average aspect ratio of 50 or more and 600 or less, and
   having a luminosity intensity Si value at measurement angles θ of 15° and 45° in a range of 1.00 or more and 15.00 or less in a luminosity measurement.
(2) The high-luminance thin-layered α-alumina powder as described in aspect (1), in which the average particle diameter is 20 µm or more and 120 µm or less, and the average thickness is 0.20 µm or more and 0.50 µm or less.
(3) The high-luminance thin-layered α-alumina powder as described in aspect (1) or (2), which contains an alkali metal (AM) in an amount of 0.2 mass% or more and 5.0 mass% or less in terms of AM₂O, and silicon (Si) in an amount of 0.3 mass% or more and 10.0 mass% or less in terms of SiO₂.
(4) The high-luminance thin-layered α-alumina powder as described in aspect (3), in which the alkali metal (AM) is sodium (Na) and/or potassium (K).
(5) The high-luminance thin-layered α-alumina powder as described in any one of aspects (1) to (4), in which the average particle diameter is 50 µm or more and 120 µm or less.
(6) The high-luminance thin-layered α-alumina powder as described in any one of aspects (1) to (5), in which the luminosity intensity Si value is in a range of 5.00 or more and 15.00 or less.
(7) A method for producing the high-luminance thin-layered α-alumina powder as described in any one of aspects (1) to (6), including:
   a step of adding an additive to a thin-layered boehmite powder and then performing mixing to prepare a raw material mixture; and
   a step of calcining the raw material mixture at a temperature in a range of 1,000°C or higher and 1,300°C or lower,
   the raw material mixture containing 0.5 mass% or more and 10.0 mass% or less of an alkali metal (AM) in terms of AM₂O, 0.1 mass% or more and 10.0 mass% or less of silicon (Si) in terms of SiO₂, and 0.1 mass% or more and 5.0 mass% or less of fluorine (F).
(8) The method as described in aspect (7), in which the thin-layered boehmite powder has an average particle diameter of 2 µm or more and 15 µm or less and an average aspect ratio of 20 or more and 60 or less.
(9) The method as described in aspect (7) or (8), in which the additive contains aluminum fluoride (AlF₃) and silicon oxide (SiO₂).
(10) The method as described in aspect (9), in which the additive further contains an oxide (AM₂O) and/or a carbonate (AM₂CO₃) of the alkali metal (AM).
(11) The method as described in aspect (7) or (8), in which the additive contains an alkali fluorosilicate (AM₂SiF₆).
(12) The method as described in any one of aspects (7) to (11), in which the alkali metal (AM) is sodium (Na) and/or potassium (K).
(13) A coating or cosmetic containing the high-luminance thin-layered α-alumina powder as described in any one of aspects (1) to (6).

### Effects of the Invention

According to the present invention, there are provided a thin-layered α-alumina powder having a large average particle diameter and a large aspect ratio and having excellent luminosity, and a method for producing the thin-layered α-alumina powder by a simple method.

### BRIEF DESCRIPTION OF THE DRAWINGS

FIG. 1 illustrates a luminosity-emerging mechanism using a thin-layered α-alumina powder;
FIG. 2 is a diagram for illustrating a luminosity evaluation method;
FIG. 3 is a diagram for illustrating the luminosity evaluation method;
FIG. 4A illustrates SEM images of a thin-layered α-alumina powder (Example 1);
FIG. 4B illustrates SEM images of a thin-layered α-alumina powder (Example 1);
FIG. 5A illustrates SEM images of a thin-layered α-alumina powder (Example 3);
FIG. 5B illustrates SEM images of a thin-layered α-alumina powder (Example 3);
FIG. 6A illustrates SEM images of the thin-layered α-alumina powder (Example 3), and
FIG. 6B illustrates SEM images of the thin-layered α-alumina powder (Example 3).

### PREFERRED MODE FOR CARRYING OUT THE INVENTION

A specific embodiment of the present invention (hereinafter, referred to as "the present embodiment") will be described. The present invention is not limited to the following embodiment, and various modifications can be made without departing from the scope of the present invention.

### <<1. High-luminance thin-layered α-alumina Powder>>

A high-luminance thin-layered α-alumina powder of the present embodiment (hereinafter, simply referred to as an "α-alumina powder" or an "alumina powder" in some cases) is a powder containing α-alumina (Al₂O₃) as a main component. The α-alumina has a trigonal corundum crystal, is excellent in heat resistance, corrosion resistance, wear resistance, and insulation properties, and has high strength and hardness. Further, the α-alumina has high light reflectance, and high luminance and whiteness. In the alumina powder of the present embodiment, such characteristics can be sufficiently utilized. In the present description, the powder means an collectivity of a large number of independent particles. In other words, a large number of particles collectively make a powder. Since the particles forming the powder are independent of each other, the powder exhibits fluidity as a whole. Most of the particles forming the powder may be independent. As long as the powder exhibits fluidity as a whole, some of the particles may be bonded to form an aggregate.

The alumina powder of the present embodiment may contain other components as long as it contains α-alumina as a main component. However, in order to take advantage of an effect based on the α-alumina, a content of the α-alumina is preferably high. The content of the α-alumina is preferably 80 mass% or more, more preferably 90 mass% or more, and further preferably 95 mass% or more. The content of the α-alumina can be evaluated by determining an amount of components in the alumina powder by X-ray fluorescence.

In the alumina powder of the present embodiment, the particles constituting the alumina powder have a thin-layered (flaky, flat, plate-like) shape. That is, each particle has a large plate surface and a small plate thickness. Specifically, the alumina powder has an average particle diameter of 20 µm or more and 200 µm or less, an average thickness of 0.20 µm or more and 2.00 µm or less, and an average aspect ratio of 50 or more and 600 or less. Here, the average particle diameter and the average thickness are number-based average values of particle diameters and thicknesses of respective particles constituting the alumina powder. The particle diameter is a major axis diameter of the particle. A longest diameter of the particle plate surface is defined as the particle diameter. The thickness is a plate thickness of the plate. The average aspect ratio is a ratio of the average particle diameter to the average thickness (average particle diameter/average thickness). The particle diameter, the thickness, and the aspect ratio can be determined by observing the particles constituting the alumina powder with a scanning electron microscope (SEM).

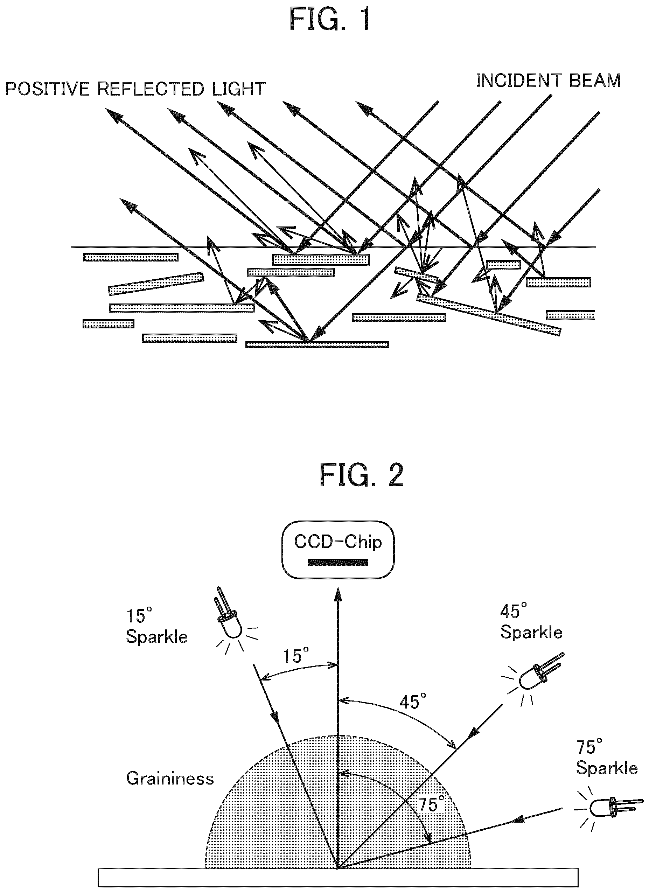

Enlarged particle diameter (major axis diameter) and aspect ratio makes it possible to enhance the luminosity of the alumina powder. This point will be described with reference to FIG. 1. As illustrated in FIG. 1, when a powder having a high aspect ratio is formed on a substrate by a method such as coating, the powder tends to be oriented such that a particle plate surface having a large area is aligned in parallel with a surface of the substrate. As the particle diameter is increased, a surface area of the particle plate surface aligned in parallel with the surface of the substrate is increased. Light incident from the outside is reflected by the particle plate surface to be emitted to the outside as reflected light. Therefore, as the particle diameter and the aspect ratio are increased, the incident light is more easily reflected by the particle plate surface, and as a result, the luminosity in visual recognition is enhanced.

As the particle diameter and the aspect ratio are increased, the luminosity of the alumina powder is enhanced. When the average particle diameter is less than 20 µm, the average thickness is more than 2.00 µm, or the average aspect ratio is less than 50, the luminosity of the alumina powder is insufficient. On the other hand, it is difficult to produce an alumina powder having an average particle diameter of more than 200 µm, an average thickness of less than 0.20 µm, or an average aspect ratio of more than 600. In addition, the strength is low and handling becomes difficult. The average particle diameter is preferably 50 µm or more and 120 µm or less, more preferably 70 µm or more and 120 µm or less, and further preferably 90 µm or more and 120 µm or less. The average thickness is preferably 0.20 µm or more and 0.40 µm or less, and more preferably 0.20 µm or more and 0.30 µm or less. Preferably, the average particle diameter is 20 µm or more and 120 µm or less, and the average thickness is 0.20 µm or more and 0.50 µm or less. The average aspect ratio is preferably 150 or more and 600 or less, and more preferably 250 or more and 600 or less.

It is not necessary that all the particles constituting the alumina powder are thin-layered. As long as the alumina powder as a whole satisfies the requirements of the average particle diameter, the average thickness, and the average aspect ratio described above, the alumina powder may contain particles having a shape other than a thin-layered shape.

In the high-luminance thin-layered α-alumina powder of the present embodiment, a luminosity intensity Si value at measurement angles θ of 15° and 45° is in a range of 1.00 or more and 15.00 or less in a luminosity measurement. Here, the luminosity intensity Si is a value determined at the time of luminosity evaluation to be described later, and is an index indicating an intensity of luminance based on bright spots of a sample (alumina powder). As the luminosity intensity Si value is increased, the luminosity of the alumina powder is enhanced. When the luminosity intensity Si value is less than 1.00, the luminosity of the alumina powder is insufficient. In addition, it is difficult to produce an alumina powder having a luminosity intensity Si value of more than 15.00. The luminosity intensity Si value is preferably 5.00 or more and 15.00 or less, and more preferably 7.00 or more and 15.00 or less.

Preferably, a luminosity area Sa value at measurement angles θ of 15° and 45° is 1.00 or more and 30.00 or less. The luminosity area Sa is a value determined at the time of the luminosity evaluation, and is an index indicating a size of each bright spot of the sample (alumina powder). The luminosity area Sa value is more preferably 10.00 or more and 30.00 or less, and further preferably 15.00 or more and 30.00 or less.

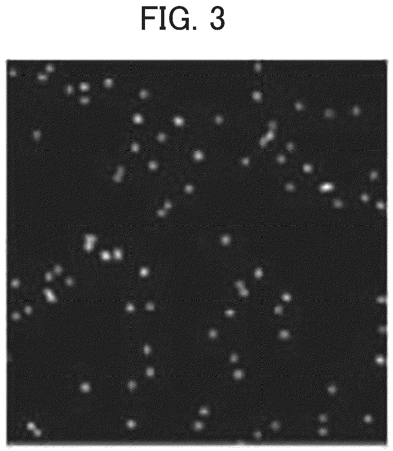

The luminosity of the alumina powder can be evaluated using a meter for multi-angle color measurement/luminosity/ particle feeling measuring device (BYK-mac i, BYK). An evaluation method will be described with reference to FIGS. 2 and 3. At the time of measurement, first, a full amount of a sample (alumina powder) is charged into a sample holder attached to a device and levelled to the top of the sample holder. Then, the sample holder into which the sample is charged is set in the device and measurement is performed. In the device, a CCD chip is provided on an extension of a vertical axis with respect to a measurement surface of the sample holder. A light source is provided at a position separated from the vertical axis via a predetermined measurement angle θ. As illustrated in FIG. 2, during measurement, light is incident from the light source toward the sample, is reflected and scattered by the measurement surface, and then is incident on the CCD chip. A light intensity of the reflected and scattered light incident on the CCD chip is measured and analyzed to acquire image data. As illustrated in FIG. 3, in the image data, a large number of bright spots based on each particle constituting the sample (alumina powder) are observed. The luminosity intensity Si value and the luminosity area Sa value can be determined by analyzing and digitizing the obtained image data.

The high-luminance thin-layered α-alumina powder of the present embodiment preferably contains an alkali metal (AM) in an amount of 0.2 mass% or more and 5.0 mass% or less in terms of AM₂O (as an equivalent amount of AM₂O), and silicon (Si) in an amount of 0.3 mass% or more and 10.0 mass% or less in terms of SiO₂ (as an equivalent amount of SiO₂). A content of the alkali metal (AM) is more preferably 0.2 mass% or more and 2.0 mass% or less, and further preferably 0.2 mass% or more and 1.0 mass% or less in terms of AM₂O. A content of the silicon (Si) is more preferably 0.5 mass% or more and 3.0 mass% or less in terms of SiO₂. Preferably, the alkali metal (AM) is sodium (Na) and/or potassium (K), that is, at least one selected from the group consisting of sodium (Na) and potassium (K). Incorporation of these components can further enhance the luminosity of the alumina powder.

As will be described later, the alkali metal (AM) and the silicon (Si) are components contained in a raw material mixture during production of the alumina powder, and become a liquid phase during calcining. When a composition of the liquid phase component is appropriately controlled, it promotes crystallization, grain growth, and surface smoothing of alumina particles. Therefore, it is possible to obtain an alumina powder having a large average particle diameter and a large aspect ratio, a smooth particle surface, and excellent luminosity.

In addition, an alkali metal oxide (AM₂O) and silicon oxide (SiO₂) contained in the alumina powder themselves have an effect of enhancing the luminosity of the alumina powder. More specifically, a liquid phase component formed in a calcination step during the production of the alumina powder is segregated as a heterophase such as glass on a surface and/or the inside of the particle in the produced alumina powder. The heterophase (glass phase or the like) has a refractive index different from that of alumina. Therefore, it is presumed that the presence of such a heterophase increases reflection and scattering of light incident from the outside, which results in enhanced luminosity.

As described above, appropriately-adjusted amounts of alkali metal (AM) and silicon (Si), as well as controlled average particle diameter and aspect ratio, make it possible to further improve the luminosity of the alumina powder. In contrast, when the amount of the alkali metal and the amount of the silicon are inappropriate, there is a concern that the luminosity is lowered.

The high-luminance thin-layered α-alumina powder of the present embodiment contains at least aluminum (Al), an alkali metal (AM), silicon (Si), and oxygen (O) as essential components (elements). However, as long as the requirements described above are satisfied, components (elements) other than the essential components (Al, AM, Si, and O) may be contained. In addition, the presence of impurities inevitably incorporated in a production process is allowed. Examples of such other components include fluorine (F). Of these, fluorine (F) is a component derived from an additive to be added during the production of the alumina powder. As will be described later, the additive to be added during the production of the alumina powder may contain fluorine (F). Most of the fluorine will be vaporized in the calcination step, but some of the fluorine may remain. An amount of the fluorine in the alumina powder is typically 0.5 mass% or less, 0.3 mass% or less, or 0.1 mass% or less.

When a large amount of other components are contained, the luminosity of the alumina powder may be impaired. Therefore, a content of the components other than the essential components (Al, AM, Si, and O) is preferably 10 mass% or less, more preferably 5 mass% or less, further preferably 1 mass% or less, particularly preferably 0.5 mass% or less, and most preferably 0.1 mass% or less. In particular, iron (Fe) has an effect of coloring the alumina powder, and accordingly, it is preferable to control a content thereof. Preferably, the content of the iron (Fe) is 0.05 mass% or less in terms of iron oxide (Fe₂O₃).

A specific surface area (S_{BET}) of the high-luminance thin-layered α-alumina powder of the present embodiment is preferably 0.1 m²/g or more and 5.0 m²/g or less, and further preferably 0.5 m²/g or more and 2.0 m²/g or less. When the specific surface area is excessively large, the particle diameter of the alumina powder becomes small, so that there is a concern that the luminosity is lowered. On the other hand, it is difficult to produce a thin-layered α-alumina powder having a small specific surface area.

The high-luminance thin-layered α-alumina powder of the present embodiment is preferably composed of a polycrystalline. In other words, the respective particles constituting the alumina powder are in a polycrystalline state. An alumina powder composed of a polycrystalline can improve its luminosity. Although details of the reason are unclear, it is presumed that a silicate (AM₂SiO₃) is formed from the essential components (AM, Si, and O), and this promotes melting and adhesion of the alumina particles to each other to cause polycrystallization.

The high-luminance thin-layered α-alumina powder of the present embodiment contains α-alumina as a main component, and thus has high chemical stability. In addition, the average particle diameter and the aspect ratio are large and the luminosity is excellent. The α-alumina powder having such characteristics is suitably used in the fields of coatings (paints) and cosmetics. However, the α-alumina powder of the present embodiment is not limited to those used for coatings and cosmetics. The α-alumina powder can be applied to known applications such as reinforcing materials and gas barrier materials to be added in plastic and a resin film, or structural members, tools, abrasives, fillers, spark plugs, insulators, electronic substrates, and refractory materials.

### <<2. Method for Producing High-luminance thin-layered α-alumina Powder>>

A method for producing the high-luminance thin-layered α-alumina powder of the present embodiment is not limited as long as the requirements described above are satisfied. However, the high-luminance thin-layered α-alumina powder is preferably produced by the following procedure. The preferred production method includes a step of adding an additive to a thin-layered boehmite powder and then performing mixing to prepare a raw material mixture (raw material mixing step), and a step of calcining the raw material mixture at a temperature in a range of 1,000°C or higher and 1,300°C or lower (calcination step). If necessary, a post-treatment step may be provided after the calcination step. The raw material mixture contains 0.5 mass% or more and 10.0 mass% or less of an alkali metal (AM) in terms of AM₂O (as an equivalent amount of AM₂O), 0.1 mass% or more and 10.0 mass% or less of silicon (Si) in terms of SiO₂ (as an equivalent amount of SiO₂), and 0.1 mass% or more and 5.0 mass% or less of fluorine (F). Each step will be described in detail below.

### <Raw Material Mixing Step>

In the raw material mixing step, an additive is added to a thin-layered boehmite powder (hereinafter, may be simply referred to as a "boehmite powder") and then mixed to prepare a raw material mixture. The thin-layered boehmite powder is a thin-layered powder containing boehmite (AlOOH), which is alumina monohydrate, as a main component, and is an aluminum source of an alumina powder. The thin-layered boehmite powder used as a raw material makes it possible to obtain an alumina powder having a large average particle diameter and a large aspect ratio. In the subsequent calcination step, boehmite is transferred to α-alumina via intermediate alumina. When the thin-layered boehmite powder is used as the raw material, a particle shape thereof is inherited, and thus a thin-layered α-alumina powder can be obtained.

A size of the boehmite powder is not particularly limited as long as the alumina powder of the present embodiment can be obtained. However, the average particle diameter is preferably 2 µm or more and 15 µm or less and an average aspect ratio is preferably 20 or more and 60 or less. By using the boehmite powder having such a size, the alumina powder of the present embodiment can be easily obtained. In addition, the production method of the boehmite powder having such a size is known and is easily available. For example, Patent Document 5 discloses a method for producing a hexagonal plate-like boehmite in which aluminum hydroxide and boric acid are hydrothermally treated after an addition of a pH adjusting agent such as sodium hydroxide. The obtained hexagonal plate-like boehmite has an outer diameter size of 0.7 µm to 15.0 µm and an aspect ratio of 40 to 100 (Claim 2, and paragraphs [0017] and [0018] of Patent Document 5).

The additive is a component that becomes a liquid phase which acts as a mineralizer in the subsequent calcination step. The additive contains at least silicon (Si) and fluorine (F), and may further contain an alkali metal (AM) as necessary. As to be described later, when the composition of the liquid phase component is appropriately controlled, it promotes crystallization, grain growth, and surface smoothing of the alumina particles. As a result, it becomes possible to obtain an alumina powder having a large average particle diameter and a large aspect ratio, a smooth particle surface, and excellent luminosity. In addition, the liquid phase component is present as a heterophase such as glass in the produced alumina powder, and has an effect of further enhancing the luminosity.

Accordingly, it is important to appropriately control a composition of the raw material mixture containing the additive in order to obtain an alumina powder having a large average particle diameter, a large aspect ratio, and excellent luminosity. Specifically, a composition of the additive is adjusted such that the raw material mixture contains 0.5 mass% or more and 10.0 mass% or less of an alkali metal (AM) in terms of AM₂O, 0.1 mass% or more and 10.0 mass% or less of silicon (Si) in terms of SiO₂, and 0.1 mass% or more and 5.0 mass% or less of fluorine (F). The composition of the raw material mixture within the range described above makes it possible to obtain an alumina powder having a large average particle diameter, a large aspect ratio, and excellent luminosity. In contrast, when the composition of the raw material mixture is not appropriate, the luminosity of the alumina powder may be lowered.

The amount of an alkali metal (AM) in the raw material mixture is more preferably 0.5 mass% or more and 5.0 mass% or less, and further preferably 0.5 mass% or more and 3.0 mass% or less in terms of AM₂O. The alkali metal is preferably sodium (Na) and/or potassium (K), that is, at least one selected from the group consisting of sodium (Na) and potassium (K). The amount of silicon (Si) in the raw material mixture is more preferably 0.5 mass% or more and 10.0 mass% or less, and further preferably 1.0 mass% or more and 5.0 mass% or less in terms of SiO₂. The amount of fluorine (F) in the raw material mixture is more preferably 0.1 mass% or more and 5.0 mass% or less, and further preferably 1.0 mass% or more and 3.0 mass% or less. When the composition of the raw material mixture is limited within the range described above, it is possible to further enhance the luminosity of the obtained alumina powder.

The additive contains at least silicon (Si) and fluorine (F). The additive may or may not contain an alkali metal (AM). A raw material boehmite powder may contain alkali metal (AM) as an impurity. When an amount of alkali metal in the boehmite powder is sufficient, the additive does not have to contain an alkali metal. However, when the amount of alkali metal in the boehmite powder is insufficient, it is preferable to add an alkali metal as an additive. Further, the additive may contain aluminum (Al). In case where the additive contains aluminum, this aluminum will be incorporated into the alumina powder in the calcination step.

As long as an alumina powder having a high aspect ratio and high luminosity can be obtained, the additive may contain a component other than alkali metal (AM), silicon (Si), fluorine (F), aluminum (Al), and oxygen (O). However, when an amount of the other component is excessively large, it may be difficult to obtain an alumina powder having high luminosity. Therefore, a content of the other component is preferably 20 mass% or less, more preferably 10 mass% or less, further preferably 5 mass% or less, and particularly preferably 1 mass% or less. The additive may not contain components other than alkali metal (AM), silicon (Si), fluorine (F), aluminum (Al), and oxygen (O) in an amount exceeding an amount of impurities.

According to a preferred embodiment, the additive may contain aluminum fluoride (AlF₃) and silicon oxide (SiO₂). When the amount of alkali metal in the boehmite powder is insufficient, the additive preferably further contains an oxide (AM₂O) and/or a carbonate (AM₂CO₃) of alkali metal (AM), that is, at least one selected from the group consisting of an oxide (AM₂O) and a carbonate (AM₂CO₃). Further, according to another preferred embodiment, the additive may contain an alkali fluorosilicate (AM₂SiF₆). An appropriate amount of such compounds (AlF₃, SiO₂, AM₂O, AM₂CO₃, and AM₂SiF₆) used as additives makes it possible to reliably prepare an alumina powder having a large average particle diameter, a large aspect ratio, and excellent luminosity. In addition, these compounds are inexpensive and easily available. Therefore, the thin-layered alumina powder can be obtained at a lower cost.

Mixing of the boehmite powder and the additive may be performed by a known method. The mixing may be performed in a dry manner or a wet manner. Dry mixing may be performed using a dry mixer such as an air blender, a V-type blender, a locking blender, a Henschel mixer, and a Nauta mixer. In the case of wet mixing, a solvent such as water may be added to the boehmite powder and the additive to form a slurry, and the obtained slurry may be mixed using a wet mixer such as a ball mill, an attritor, and a bead mill.

### <Calcination Step>

In the calcination step, the raw material mixture containing the thin-layered boehmite powder and the additive is calcined at a temperature in a range of 1,000°C or higher and 1,300°C or lower to obtain a calcined product. During calcining, a boehmite (AlOOH) is transferred to α-alumina (Al₂O₃), which is a high-temperature stable phase, through an intermediate alumina such as γ-Al₂O₃, which is a metastable phase. A particle shape is maintained during the transfer, and accordingly, a thin-layered α-alumina (Al₂O₃) can be obtained. In addition, alkali metal (AM), silicon (Si), and fluorine (F) in the raw material mixture become a liquid phase in an oxide state during calcining, and act as a mineralizer which promotes crystallization of the α-alumina. That is, at the time of transferring from the intermediate alumina to the α-Al₂O₃, a crystal structure changes from a cubic close-packed structure to a hexagonal close-packed structure, which rearrange oxygen in the crystal accordingly. Therefore, a certain amount of thermal energy should be applied for the transfer to α-Al₂O₃. The mineralizer added promotes crystallization of the crystal structure (corundum structure) of the α-Al₂O₃, and thus lowers the transfer temperature.

In addition, the mineralizer components (AM, Si, and F) that have become a liquid phase during calcining cover a surface of the alumina particle, and some of them enter the inside of the particle. The liquid phase components have a function of promoting diffusion and growth of particles. That is, some of aluminum atoms (Al) are dissolved in the liquid phase from the surface of the alumina particle, and the dissolved aluminum will be deposited at another location on the surface of the particle. This causes the growth of the alumina particle. In this case, the growth direction of the alumina particle varies and the growth rate changes depending on the composition of the liquid phase components. Therefore, when the composition of the liquid phase components is appropriately controlled, the growth of the alumina particle along a plate surface direction proceeds remarkably. The liquid phase components also have an effect of smoothing the plate surface of the alumina particle. In a case where the surface of the alumina particle has irregularities, the irregularities are preferentially diffused into the liquid phase, so that the irregularities disappear. Therefore, appropriately-controlled composition of the liquid phase components makes it possible to obtain an alumina powder having a large average particle diameter, a large aspect ratio, a smooth particle surface, and excellent luminosity.

When a calcining temperature is lower than 1,000°C, the transfer to α-alumina and the grain growth may be insufficient. Therefore, it may be difficult to obtain an α-alumina powder having excellent luminosity. On the other hand, when the calcining temperature is higher than 1,300°C, particles may be sintered to form a strong sintered mass. In addition, energy consumption for calcining becomes excessive, which may lead to an increase in production cost. The calcining temperature is preferably 1,000°C or higher and 1,200°C or lower, and more preferably 1,000°C or higher and 1,100°C or lower. A calcining time is preferably 10 hours or more and 20 hours or less. This makes it possible to reliably ensure the transfer to α-alumina and the grain growth while preventing excessive sintering of the particles. A calcining furnace is not limited as long as a desired alumina powder can be obtained. However, from the viewpoint of effectively achieving the effects of the additive during calcining, preferable is a stationary furnace that can perform calcining using a sealed calcining container.

### <Post-treatment>

If necessary, the calcined product obtained through the calcination step may be subjected to a post-treatment such as a soda removal treatment, a pulverization treatment, and/or a classification treatment. In the soda removal treatment, excess alkali metal component, which adheres to a surface of the calcined product, is removed. Thus, an amount of the alkali metal in the α-alumina powder to be finally obtained can be adjusted. The alkali metal component on the particle surface can be removed, for example, by washing the calcined product with water and filtering the calcined product. In the pulverization treatment, a slight amount of mechanical energy is applied to the calcined product to unbind the aggregated particles formed during calcining. The pulverization treatment can be performed in a dry manner or a wet manner using a pulverizer such as a pot mill, a pin mill, and/or a jaw crusher. In the classification treatment, particles are classified according to the size to obtain an alumina powder having a desired particle size. The classification treatment can be performed by a method such as sieving, air flow classification, elutriation, and/or centrifugation. The post-treatment may be performed as necessary. The post-treatment may be omitted as long as the desired alumina powder is obtained after calcining.

In this manner, the thin-layered α-alumina powder of the present embodiment can be prepared. The obtained alumina powder has a high average particle diameter, a high aspect ratio, and excellent luminosity. Therefore, the alumina powder can be suitably used in various applications including coatings and cosmetics.

### <<3. Coating and Cosmetic>>

The coating (paint) or cosmetic of the present embodiment contains the high-luminance thin-layered α-alumina powder described above. The coating or cosmetic may contain the alumina powder in a single form or in a surface-treated form. A surface treatment may be performed by providing a surface treatment agent such as a silicon-based compound, an alkylsilane-based compound, and/or a fluorine-based compound on the particle surface of the thin-layered α-alumina powder. The coating and the cosmetic may contain a solvent or a resin in addition to the thin-layered α-alumina powder. The solvent may be water-based or non-aqueous. Further, the coating and the cosmetic may contain known additive components such as an oil agent, a pigment other than the thin-layered α-alumina powder, a filler, a surfactant, a viscosity modifier, a preservative, a flavor, a humectant, a physiologically active component, salts, a chelating agent, a neutralizing agent, and/or a pH adjuster.

### EXAMPLES

The present invention will be described in more detail with reference to the following examples. However, the present invention is not limited to the following examples.

### (1) Preparation of alumina powder

### [Example 1]

### <Raw Material Mixing Step>

As an alumina source, a thin-layered boehmite powder (BMF-1160, KAWAI LIME INDUSTRY CO., LTD.) was prepared. As an additive, aluminum fluoride (AlF₃, DO-FLUORIDE CHEMICALS CO., LTD.) and silicon oxide (SiO₂, SNOWMARK silica stone SP-3, Marukama Kamado, Inc.) were prepared. Properties of the prepared thin-layered boehmite powder are shown in Table 1 below.

Next, 50 g of the prepared thin-layered boehmite powder, 1.2 g of the prepared aluminum fluoride (AlF₃), and 0.4 g of the prepared silicon oxide (SiO₂) were mixed to obtain a raw material mixture. The mixing was performed by putting the raw materials into a bag and shaking the bag. The obtained raw material mixture contained 0.7 mass% of sodium (Na) in terms of Na₂O, 0.8 mass% of silicon (Si) in terms of SiO₂, and 1.6 mass% of fluorine (F).

### <Calcination Step>

The obtained raw material mixture was charged into a crucible (SSA-H 500 mL, Nikkato Corporation), and the crucible was closed with a dedicated lid. The crucible had a composition of 95% of Al₂O₃ and 3% of SiO₂. Next, the crucible with the lid was placed inside a calcining furnace (BSH-3570, box-type Siliconit electric furnace, Siliconit Co., Ltd.) and calcined in air. At the time of calcining, a temperature was increased to 1,100°C at a temperature increase rate of 100°C/hour, and then kept at 1,100°C for 10 hours, followed by natural cooling. After the temperature in the furnace was completely lowered, the crucible was taken out of the furnace, and a calcined product (alumina) was collected.

### <Soda Removal Step>

The obtained calcined product (alumina) was subjected to a soda removal treatment. Specifically, 340 g of the calcined product was introduced into 7 L of pure water and then mixed to form a slurry. Next, the obtained slurry was filtered to obtain an alumina residue. Thus, sodium adhering to the calcined product was removed.

### <Pulverization Step>

The obtained alumina residue was subjected to a pulverization treatment. Specifically, pure water was added to the alumina residue again, followed by stirring to form a slurry. The obtained slurry was put into a pot mill container having an internal volume of 10 L together with 15 kg of zirconia beads having a diameter of 1 mm. The pot mill container was then rotated at a rotation speed of 60 rpm for 3 hours. Next, the slurry was taken out from the pot mill container and filtered to obtain a pulverized product (alumina). Then, the obtained pulverized product was dried overnight at 105°C using a dryer.

### <Classification Step>

The obtained pulverized product was subjected to a classification treatment. Specifically, pure water was added to the pulverized product, followed by stirring to form a slurry. Next, the obtained slurry was sieved using a resin net gyro shifter having a mesh size of 25 µm (GS-A1H, TOKUJU Co., LTD). The sieving was performed under a condition of a rotation speed of 60 rpm to remove coarse particles having a size of more than 25 µm. Thereafter, a sieve-net-through product from which the coarse particles had been removed was filtered using a SUS net having a mesh size of 12 µm to remove fine particles having a size of less than 12 µm. In this manner, an alumina powder was prepared.

### [Example 2]

In the raw material mixing step, a raw material mixture was obtained by mixing 50 g of a thin-layered boehmite powder (BMF-1160, KAWAI LIME INDUSTRY CO., LTD.), 1.2 g of aluminum fluoride (AlF₃), and 0.6 g of silicon oxide (SiO₂). The obtained raw material mixture contained 0.7 mass% of sodium (Na) in terms of Na₂O, 1.2 mass% of silicon (Si) in terms of SiO₂, and 1.6 mass% of fluorine (F). A classification treatment was performed by the following procedure without performing a soda removal treatment and a pulverization treatment. An alumina powder was prepared in the same manner as in Example 1 except for the above.

### <Classification Step>

The calcined product (alumina) obtained in the calcination step was subjected to elutriation classification using an elutriation tube and particle size adjustment using a SUS net to remove extremely thick particles and fine particles. Specifically, pure water was added to 300 g of the calcined product, followed by stirring to obtain a slurry having a concentration of 100 g/L. The obtained slurry was charged into a classifier (elutriation tube), and pure water was continuously introduced thereon at a flow rate of 1 L/min for 3 hours. Thereafter, the slurry containing a precipitate was collected from the classifier. Next, by sequentially using a SUS net having a mesh size of 63 µm and a SUS net having a mesh size of 45 µm, manual sieving was performed to remove fine particles having a size of less than 45 µm from the collected slurry. A residue obtained after the manual sieving was dried overnight using a dryer.

### [Example 3]

In the raw material mixing step, a raw material mixture was obtained by mixing 50 g of a thin-layered boehmite powder (BMF-1160, KAWAI LIME INDUSTRY CO., LTD.), 1.2 g of aluminum fluoride (AlF₃), and 0.8 g of silicon oxide (SiO₂). The obtained raw material mixture contained 0.7 mass% of sodium (Na) in terms of Na₂O, 1.6 mass% of silicon (Si) in terms of SiO₂, and 1.6 mass% of fluorine (F). A classification treatment was performed by the following procedure without performing a soda removal treatment and a pulverization treatment. An alumina powder was prepared in the same manner as in Example 1 except for the above.

### <Classification Step>

The calcined product (alumina) obtained in the calcination step was subjected to elutriation classification using an elutriation tube and particle size adjustment using a SUS net to remove extremely thick particles and fine particles. Specifically, pure water was added to 300 g of the calcined product, followed by stirring to obtain a slurry having a concentration of 100 g/L. The obtained slurry was charged into a classifier (elutriation tube), and pure water was continuously introduced thereon at a flow rate of 1 L/min for 3 hours. Thereafter, the slurry containing a precipitate was collected from the classifier. Next, by using a SUS net having a mesh size of 45 µm, manual sieving was performed to remove fine particles having a size of less than 45 µm from the collected slurry. A residue obtained after the manual sieving was dried overnight using a dryer.

### [Example 4]

In the raw material mixing step, a raw material mixture was obtained by mixing 50 g of a thin-layered boehmite powder (BMF-1160, KAWAI LIME INDUSTRY CO., LTD.), 1.2 g of aluminum fluoride (AlF₃), 0.8 g of silicon oxide (SiO₂), and 0.08 g of sodium carbonate (Na₂CO₃). The obtained raw material mixture contained 0.6 mass% of sodium (Na) in terms of Na₂O, 1.6 mass% of silicon (Si) in terms of SiO₂, and 1.6 mass% of fluorine (F). A classification treatment was performed by the following procedure without performing a soda removal treatment and a pulverization treatment. An alumina powder was prepared in the same manner as in Example 1 except for the above.

### <Classification Step>

The calcined product (alumina) obtained in the calcination step was subjected to elutriation classification using an elutriation tube and particle size adjustment using a SUS net to remove extremely thick particles and fine particles. Specifically, pure water was added to 300 g of the calcined product, followed by stirring to obtain a slurry having a concentration of 100 g/L. The obtained slurry was charged into a classifier (elutriation tube), and pure water was continuously introduced thereon at a flow rate of 1 L/min for 3 hours. Thereafter, the slurry containing a precipitate was collected from the classifier. Next, by using a SUS net having a mesh size of 63 µm, manual sieving was performed to remove fine particles having a size of less than 63 µm from the collected slurry. A residue obtained after the manual sieving was dried overnight using a dryer.

### [Example 5]

In the raw material mixing step, a raw material mixture was obtained by mixing 50 g of a thin-layered boehmite powder (BMF-1160, KAWAI LIME INDUSTRY CO., LTD.), and 2.5 g of sodium fluorosilicate (Na₂SiF₆). The obtained raw material mixture contained 0.7 mass% of sodium (Na) in terms of Na₂O, 1.6 mass% of silicon (Si) in terms of SiO₂, and 2.8 mass% of fluorine (F). A soda removal treatment, a pulverization treatment, and a classification treatment were not performed. An alumina powder was prepared in the same manner as in Example 1 except for the above.

### [Example 6]

As an alumina source, a thin-layered boehmite powder (BMF-920, KAWAI LIME INDUSTRY CO., LTD.) was used. The obtained raw material mixture contained 0.6 mass% of sodium (Na) in terms of Na₂O, 1.6 mass% of silicon (Si) in terms of SiO₂, and 1.6 mass% of fluorine (F). A soda removal treatment, a pulverization treatment, and a classification treatment were not performed. An alumina powder was prepared in the same manner as in Example 3 except for the above.

### [Example 7]

In the raw material mixing step, a raw material mixture was obtained by mixing 50 g of a thin-layered boehmite powder (BMF-1160, KAWAI LIME INDUSTRY CO., LTD.), 1.2 g of aluminum fluoride (AlF₃), 2.4 g of silicon oxide (SiO₂), and 1.8 g of sodium carbonate (Na₂CO₃). The obtained raw material mixture contained 0.7 mass% of sodium (Na) in terms of Na₂O, 4.4 mass% of silicon (Si) in terms of SiO₂, and 1.5 mass% of fluorine (F). A soda removal treatment, a pulverization treatment, and a classification treatment were not performed. An alumina powder was prepared in the same manner as in Example 1 except for the above.

### [Example 8]

As an alumina source, a thin-layered boehmite powder (BMF-520, KAWAI LIME INDUSTRY CO., LTD.) was used. In the raw material mixing step, a raw material mixture was obtained by mixing 50 g of a thin-layered boehmite powder (BMF-520, KAWAI LIME INDUSTRY CO., LTD.), 1.2 g of aluminum fluoride (AlF₃), 4.8 g of silicon oxide (SiO₂), and 1.2 g of sodium carbonate (Na₂CO₃). The obtained raw material mixture contained 0.6 mass% of sodium (Na) in terms of Na₂O, 8.4 mass% of silicon (Si) in terms of SiO₂, and 1.4 mass% of fluorine (F). A soda removal treatment, a pulverization treatment, and a classification treatment were not performed. An alumina powder was prepared in the same manner as in Example 1 except for the above.

### [Example 9]

### <Raw Material Mixing Step>

As an alumina source, a thin-layered boehmite powder (BMF-520, KAWAI LIME INDUSTRY CO., LTD.) was used. In the raw material mixing step, a thin-layered boehmite powder (BMF-520, KAWAI LIME INDUSTRY CO., LTD.) and sodium fluorosilicate (Na₂SiF₆) were mixed to obtain a raw material mixture containing 3.8 mass% of Na₂SiF₆.

### <Calcination Step>

The obtained raw material mixture was charged into a sagger (KR-4A, 300 mm × 300 mm × 100 mH, Acoh Ceramic Co., Ltd.), and the sagger was closed and sealed with a dedicated lid. Next, the sealed sagger was placed inside a calcining furnace (BSH-3570, box-type Siliconit electric furnace, Siliconit Co., Ltd.) and calcined in air. At the time of calcining, a temperature was increased to 1,100°C at a temperature increase rate of 100°C/hour, and then kept at 1,100°C for 10 hours, followed by natural cooling. After the temperature in the furnace was completely lowered, the sagger was taken out of the furnace, and a calcined product (alumina) was collected.

The collected calcined product (alumina) was used as an alumina powder. In this case, a soda removal treatment, a pulverization treatment, and a classification treatment were not performed.

### [Example 10]

### <Raw Material Mixing Step>

As an alumina source, a thin-layered boehmite powder (BMF-920, KAWAI LIME INDUSTRY CO., LTD.) was used. In the raw material mixing step, a thin-layered boehmite powder (BMF-920, KAWAI LIME INDUSTRY CO., LTD.) and sodium fluorosilicate (Na₂SiF₆) were mixed to obtain a raw material mixture containing 1.3 mass% of Na₂SiF₆.

### <Calcination Step>

A shuttle kiln (0.6 m³ shuttle kiln (under upper system), Takasago Industry Co., Ltd.) was used as a calcining furnace. A calcined product (alumina) was obtained by performing calcining under the same condition as in Example 9 except for the above.

### <Pulverization Step>

The obtained calcined product (alumina) was subjected to a pulverization treatment. Specifically, pure water was added to the calcined product, followed by stirring to form a slurry. The obtained slurry was put into a pot mill container having an internal volume of 10 L together with 15 kg of zirconia beads having a diameter of 1 mm. The pot mill container was then rotated at a rotation speed of 60 rpm for 3 hours. Next, the slurry was taken out from the pot mill container to obtain a pulverized product (alumina).

### <Classification Step>

The pulverized product (alumina) obtained in the pulverization step was subjected to elutriation classification using an elutriation tube and particle size adjustment using a SUS net to remove extremely thick particles and fine particles. Specifically, the slurry containing the pulverized product was charged into a classifier (elutriation tube), and pure water was continuously introduced thereon at a flow rate of 1 L/min for 3 hours. Thereafter, the slurry containing a precipitate was collected from the classifier. Next, by sequentially using a SUS net having a mesh size of 108 µm and a SUS net having a mesh size of 45 µm, manual sieving was performed to remove fine particles having a size of less than 45 µm from the collected slurry. A residue obtained after the manual sieving was dried overnight using a dryer.

### [Example 11]

### <Raw Material Mixing Step>

As an alumina source, a thin-layered boehmite powder (BMF-920, KAWAI LIME INDUSTRY CO., LTD.) was used. In the raw material mixing step, a thin-layered boehmite powder (BMF-920, KAWAI LIME INDUSTRY CO., LTD.), aluminum fluoride (AlF₃), silicon oxide (SiO₂), and sodium carbonate (Na₂CO₃) were mixed to obtain a raw material mixture containing 2.7% of AlF₃, 1.0 mass% of SiO₂, and 1.7 mass% of Na₂CO₃.

### <Calcination Step>

The obtained raw material mixture was calcined, and the calcined product (alumina) was collected. The calcining was performed under the same condition as in Example 10 except that a holding time was 20 hours.

The collected calcined product (alumina) was used as an alumina powder. In this case, a soda removal treatment, a pulverization treatment, and a classification treatment were not performed.

### [Example 12]

Raw materials were mixed to obtain a raw material mixture containing 3.1 mass% of sodium fluorosilicate (Na₂SiF₆). A holding time during calcining was 20 hours. An alumina powder was prepared in the same manner as in Example 10 except for the above.

### [Comparative Example 1]

### <Raw Material Mixing Step>

As an alumina source, an alumina powder having a BET specific surface area of 0.8 m²/g to 1.2 m²/g produced by rotary kiln calcining was prepared. Properties of the prepared alumina powder are shown in Table 1 below.

Next, the alumina powder and silicon oxide (SiO₂) were mixed to obtain a raw material mixture. The silicon oxide was added so as to be 0.3 mass% with respect to the alumina powder. The silicon oxide changes sodium (Na) in the alumina powder into a sodalite form in a melting step to be described later. Since sodalite can be removed by an acid washing step, an amount of a sodium component in alumina can be reduced by changing it to the sodalite form.

### <Melting Step>

The obtained raw material mixture was subjected to an arc discharge treatment in an electric arc furnace for 8 hours to 9 hours. During the treatment, the raw material mixture was melted into molten substance. The temperature of the molten substance was higher than 2,000°C. After melting, the molten substance was cooled to obtain a solidified mass.

### <Crushing and Grinding>

The obtained solidified mass was broken into head-sized pieces by using a powerman, and then subjected to sieving by using a trommel (25 mm × 25 mm sieve). Then, a mass larger than 25 mm remaining on a mesh was collected and ground until particles of several tens of µm to several mm in size were obtained.

### <Acid Washing>

The obtained ground product was washed with a 4% sulfuric acid solution to wash out the sodalite formed during the melting step. The treated product after the washing was filtered using a horizontal filter, dried using a kiln, and then sieved to obtain an electrofused alumina having an average particle diameter of 250 µm to 350 µm.

### <Grinding>

The obtained electrofused alumina was ground using a continuous vibration mill to obtain an alumina powder. The average particle diameter of the obtained alumina powder was 18 µm.

### [Comparative Example 2]

As an alumina source, aluminum hydroxide A (Al(OH)₃) produced by the Bayer process was prepared. Properties of the prepared aluminum hydroxide A are shown in Table 1 below.

### <Calcining>

The prepared aluminum hydroxide A was charged in a fixed amount into a raw material charging unit of a rotary kiln calcining equipment. Adhering moisture of the aluminum hydroxide A was removed in a pre-drying unit of the calcining equipment, and then aluminum fluoride (AlF₃) was added in a fixed amount. Thereafter, the aluminum hydroxide A to which the aluminum fluoride had been added was calcined into a calcined product. The calcining was performed under a condition of 1,280°C to 1,380°C for 2 hours. In this manner, an alumina powder was obtained.

### [Comparative Example 3]

As an alumina source, imported aluminum hydroxide B (Al(OH)₃) was used. Properties of the aluminum hydroxide B used are shown in Table 1 below. An alumina powder was prepared in the same manner as in Comparative Example 2 except for the above.

### [Comparative Example 4]

In the raw material mixing step, 50 g of a thin-layered boehmite powder (BMF-1160, KAWAI LIME INDUSTRY CO., LTD.) and 1.2 g of aluminum fluoride (AlF₃) were mixed, but silicon oxide (SiO₂) was not added. The obtained raw material mixture contained 0.7 mass% of sodium (Na) in terms of Na₂O, 0.02 mass% of silicon (Si) in terms of SiO₂, and 1.6 mass% of fluorine (F). In addition, a soda removal treatment, a pulverization treatment, and a classification treatment were not performed. An alumina powder was prepared in the same manner as in Example 1 except for the above.

Production conditions of the alumina powders of Examples 1 to 12 and Comparative Examples 1 to 4 are collectively shown in Tables 2-1. 2-2 and 3 below.

**[Table 1]**

| Table 1 Properties of alumina source | | | | | | |
|---|---|---|---|---|---|---|
| | Scaly boehmite powder | | | Alumina | Aluminum hydroxide A | Aluminum hydroxide B |
| | BMF-1160 | BMF-920 | BMF-520 | | | |
| Na₂O (mass%) | 0.7 | | 0.6 | 0.3 | 0.2 | 0.3 |
| SiO₂ (mass%) | 0.02 | 0.03 | 0.03 | 0.03 | <0.01 (0.003) | 0.01 |
| Fe₂O₃ (mass%) | 0.01 | 0.01 | 0.01 | 0.02 | 0.01 | 0.01 |
| (m²/g) S_{BET} | 2.7 to 3.9 | 2.6 | 4.3 | 0.8 to 1.2 | - | - |
| Average particle diameter (µm) | 9.5 to 9.8^{Note 1} | 10.7^{Note 1} | 4.3^{Note 1} | 40 to 100^{Note 2} | 50 to 70^{Note 2} | 100 to 120^{Note 2} |
| Average aspect ratio | 60 | 20 | 20 | - | - | - |

| | | | | | | |
|---|---|---|---|---|---|---|
| Note 1) A value measured by a laser diffraction scattering type particle diameter distribution measuring device (Microtrac 3300EX II, Nikkiso Co., Ltd.). Note 2) A value measured by a laser diffraction scattering type particle diameter distribution measuring device (Microtrac 9320HRA, Nikkiso Co., Ltd.). Note 3) "-" indicates not measured. | | | | | | |

**[Table 2-1]**

| Table 2-1 Production conditions of sample | | | | | | | | | |
|---|---|---|---|---|---|---|---|---|---|
| | | Example 1 | Example 2 | Example 3 | Example 4 | Example 5 | Example 6 | Example 7 | Example 8 |
| Alumina source | Type | Scaly boehmite powder | | | | | | | |
| | Added amount (g) | 50 | 50 | 50 | 50 | 50 | 50 | 50 | 50 |
| Added amount of additive (g) | AlF₃ | 1.2 | 1.2 | 1.2 | 1.2 | - | 1.2 | 1.2 | 1.2 |
| | SiO₂ | 0.4 | 0. 6 | 0.8 | 0.8 | - | 0.8 | 2.4 | 4.8 |
| | Na₂CO₃ | - | - | - | 0.08 | - | - | 1.8 | 1.2 |
| | Na₂SiF₆ | - | - | - | - | 2.5 | - | - | |
| Calcining | Temperature (°C) | 1100 | 1100 | 1100 | 1100 | 1100 | 1100 | 1100 | 1100 |
| | Time (h) | 10 | 10 | 10 | 10 | 10 | 10 | 10 | 10 |
| Post-treatment | | Soda removal Pulverization Coarse particle removal Fine particle removal | Elutriation classification Coarse particle removal Fine particle removal | Elutriation classification Fine particle removal | Elutriation classification Fine particle removal | - | - | - | - |

**[Table 2-2]**

| Table 2-2 Production conditions of sample | | | | | |
|---|---|---|---|---|---|
| | | Comparative Example 1 | Comparative Example 2 | Comparative Example 3 | Comparative Example 4 |
| Alumina source | Type | Alumina | Aluminum hydroxide A | Aluminum hydroxide B | Scaly boehmite powder |
| | Added amount (g) | - | - | - | 50 |
| Added amount of additive (g) | AlF₃ | - | - | - | 1.2 |
| | SiO₂ | - | - | - | - |
| | Na₂CO₃ | - | - | - | - |
| | Na₂SiF₆ | - | - | - | - |
| Calcining | Temperature (°C) | More than 2,000 | 1280 to 1320 | 1270 to 1360 | 1100 |
| | Time (h) | 8 to 9 | 2 | 2 | 10 |
| Post-treatment | | Crushing Grinding Acid washing Sieving Grinding | - | - | - |

**[Table 3]**

| Table 3 Production conditions of sample | | | | | |
|---|---|---|---|---|---|
| | | Example 9 | Example 10 | Example 11 | Example 12 |
| Alumina source | Type | Scaly boehmite powder | | | |
| | Product number | BMF-520 | BMF-920 | BMF-920 | BMF-920 |
| Added amount of additive (mass%) | AlF₃ | - | - | 2.7 | - |
| | SiO₂ | - | - | 1.0 | - |
| | Na₂CO₃ | - | - | 1.7 | - |
| | Na₂SiF₆ | 3.8 | 1.3 | - | 3.1 |
| Calcining | Temperature increase rate (°C/h) | 100 | 100 | 100 | 100 |
| | Temperature (°C) | 1100 | 1100 | 1100 | 1100 |
| | Time (h) | 10 | 10 | 20 | 20 |
| | Furnace used | Electric furnace | Shuttle kiln | Shuttle kiln | Shuttle kiln |
| | Container | Sagger | Sagger | Sagger | Sagger |
| Post-treatment | | - | Pulverization Elutriation classification Coarse particle removal Fine particle removal | - | Pulverization Elutriation classification Coarse particle removal Fine particle removal |

### (2) Evaluation

Various properties of the alumina powders obtained in Examples 1 to 12 and Comparative Examples 1 to 4 were evaluated as follows.

### <Average Particle Diameter and Thickness>

Evaluation was performed using a scanning electron microscope (SEM, JSM-F100, JEOL Ltd.) and image analysis software (ImageJ). Specifically, an SEM image of the obtained alumina powder was captured, and a particle diameter and a thickness were measured by evaluating particles shown in the image by the ImageJ. An average particle diameter was an average value for 80 particles, and an average thickness was an average value for 30 particles.

### <Crystalline Phase>

A crystalline phase was evaluated using an X-ray diffractometer (RINT UltimaIII, Rigaku Corporation). Specifically, first, the obtained alumina powder was placed on a dedicated sample plate, and lightly pushed and spread to a size of 20 mm × 20 mm × 0.5 mm to prepare a measurement sample. Next, an X-ray diffraction pattern of the measurement sample was evaluated using the X-ray diffractometer.

### <Component Analysis>

In a component analysis, evaluation was performed using a scanning fluorescent X-ray analyzer (ZSX PrimusIV, Rigaku Corporation). Specifically, first, the obtained alumina powder was placed in a platinum crucible and set in a high-frequency melting device. Thereafter, the alumina powder was preheated at 700°C for 60 seconds and then subjected to a shaking treatment at 1,200°C for 120 seconds to prepare a glass-beaded sample. Next, the glass-beaded sample was evaluated using a scanning X-ray analyzer. Obtained data was compared with data of a standard sample for a calibration curve to obtain evaluation results.

### <Specific Surface Area>

A specific surface area was evaluated by a N₂ gas adsorption method using a specific surface area manual measuring device (FlowSorbIII 2305 type, Micromeritics Instrument Corp.).

### <Luminosity Evaluation>

The luminosity of the alumina powder was evaluated using a polygonal colorimeter (BYK-mac i, measurement opening diameter: 12 mm, BYK-Gardner). Specifically, an alumina powder was charged into an opening (35 mm Φ × 5 mm H) of a sample holder to prepare a measurement sample and levelled to the top of the sample holder. The measurement sample was then set in the colorimeter to perform measurement. During the measurement, a CCD chip was disposed on an axis perpendicular to a measurement surface of the sample holder, and light was irradiated from a direction at an angle of 15°, 45°, or 75° from the perpendicular axis, and an image was captured by the CCD chip. Next, the obtained image was analyzed by an image analysis algorithm, and a luminosity Si value and a luminosity area Sa value at each measurement angle (15°, 45°, and 75°) were determined.

### (3) Evaluation Results

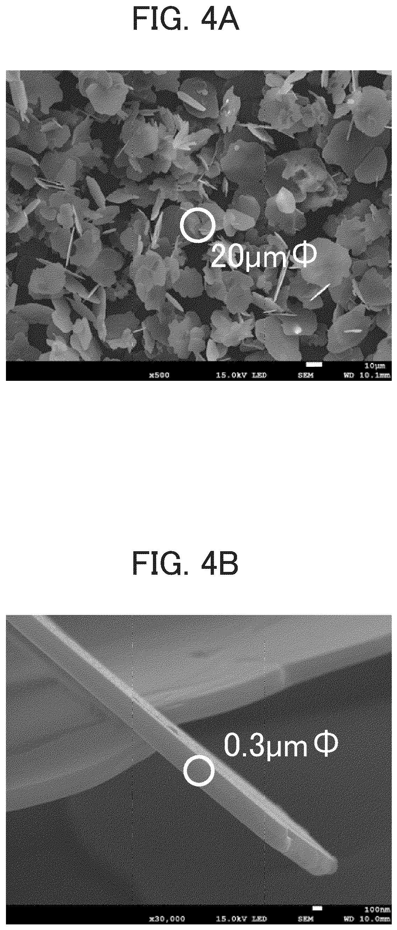

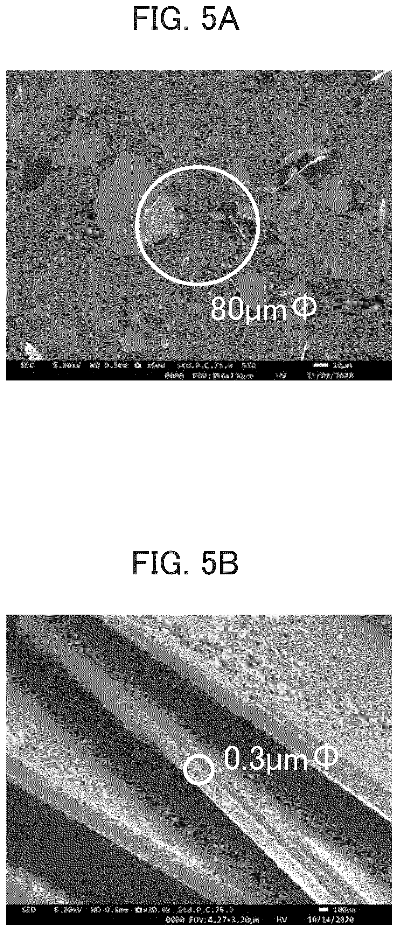

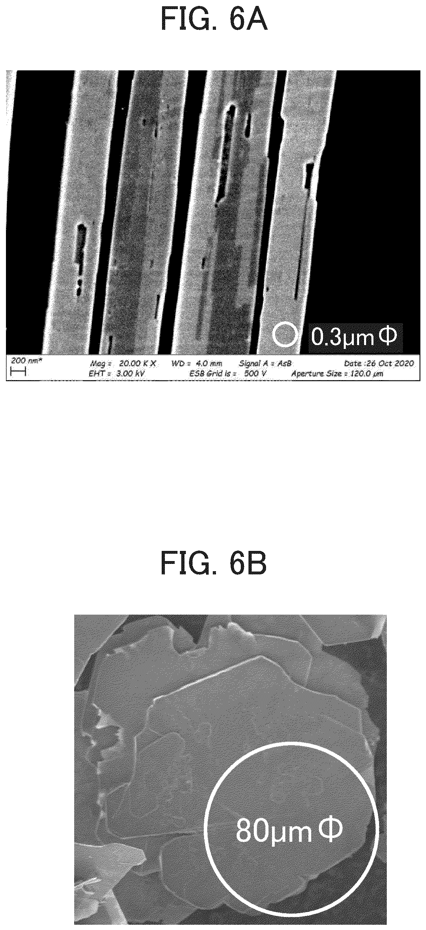

Evaluation results obtained for the alumina powders obtained in Examples 1 to 12 and Comparative Examples 1 to 4 are summarized in Tables 4-1 and 4-2. An SEM image of the alumina powder obtained in Example 1 is illustrated in FIGS. 4A and 4B, and an SEM image of the alumina powder obtained in Example 3 is illustrated in FIGS. 5A, 5B, 6A, and 6B.

As shown in Tables 4-1 and 4-2, the alumina powders of Examples 1 to 12, which were prepared using the thin-layered boehmite powder and the additives having a predetermined composition as raw materials, have an average particle diameter, an average thickness, and an average aspect ratio within the predetermined ranges. In addition, the amount of sodium oxide (Na₂O) and the amount of silicon oxide (SiO₂) satisfies the ranges specified in the present embodiment, and the luminosity intensity Si value is high. In particular, as can be seen from the results of the samples of Examples 1 to 3, as the average particle diameter is increased, the luminosity intensity Si value is increased.

From the SEM images in FIGS. 4A, 4B, 5A, and 5B, it was found that the alumina powders of Examples 1 and 3 have a thin-layered shape having a large plate surface and a small plate thickness, and the plate surface has a smooth surface. From a cross-sectional SEM image and a SEM enlarged image in FIGS. 6A and 6B, it was found that the alumina powder of Example 3 has a polycrystalline structure.

On the other hand, in Comparative Examples 1 to 3 in which alumina or aluminum hydroxide was used as a raw material, a flat alumina powder cannot be obtained. In addition, the alumina powders of Comparative Examples 1 to 3 have a small luminosity intensity Si value. The alumina powder of Comparative Example 4, which was obtained by using the thin-layered boehmite powder as a raw material and to which silicon oxide (SiO₂) was not added, has a small average particle diameter and a small aspect ratio, and has a small luminosity intensity Si value.

Based on the above results, it was found that the thin-layered α-alumina powder of the present embodiment has both a large average particle diameter and a large aspect ratio and is excellent in luminosity.

**[Table 4-1]**

| Table 4-1 Properties of alumina powder | | | | | | | | | | |
|---|---|---|---|---|---|---|---|---|---|---|
| | | Example 1 | Example 2 | Example 3 | Example 4 | Example 5 | Example 6 | Example 7 | Example 8 | Example 9 |
| Average particle diameter^{Note 1} (µm) | | 24 | 57 | 83 | 95 | 93 | 75 | 101 | 42 | 187 |
| Average particle diameter^{Note 2} (µm) | | 22 | 42 | 66 | 93 | 87 | - | - | - | - |
| Average thickness ^{Note 2} (µ | | 0.28 | 0.27 | 0.27 | 0.36 | 0.31 | 0.26 | 0.35 | 0.31 | 0.97 |
| Average aspect ratio | | 86 | 211 | 307 | 264 | 300 | 288 | 289 | 135 | 193 |
| Crystalline phase | | Corundum | Corundum | Corundum | Corundum | Corundum | Corundum | Corundum | Corundum | Corundum |
| Na₂O (mass%) | | 0.4 | 0.4 | 0.4 | 0.3 | 1.6 | 0.6 | 4.1 | 2.6 | 0.6 |
| SiO₂ (mass%) | | 0.6 | 0.7 | 1.6 | 1.2 | 1.9 | 1.4 | 5.8 | 8.5 | 0.9 |
| Fe₂O₃ (mass%) | | 0.02 | 0.02 | 0.02 | 0.02 | 0.02 | 0.02 | 0.02 | 0.02 | 0.02 |
| F (mass%) | | <0.1 | <0.1 | <0.1 | <0.1 | 0.3 | <0.1 | 0.1 | 0.1 | - |
| LOI (mass%) | | 0.2 | 0.2 | 0.2 | 0.2 | 0.2 | 0.2 | 0.2 | 0.1 | - |
| (m²/g) S_{BET} | | 1.5 | 1.3 | 1.2 | 1.0 | 1.3 | 1.0 | 0.9 | 0.9 | 0.2 |
| Luminosity intensity Si | 15° | 1.59 | 2.36 | 5.21 | - | - | - | - | - | - |
| | 45° | 1.70 | 3.12 | 8.90 | - | - | - | - | - | - |
| | 75° | 2.45 | 0.46 | 0.84 | - | - | - | - | - | - |
| Luminosity area Sa | 15° | 3.29 | 9.54 | 19.25 | - | - | - | - | - | - |
| | 45° | 2.58 | 8.44 | 19.10 | - | - | - | - | - | - |
| | 75° | 8.86 | 2.67 | 13.33 | - | - | - | - | - | - |

| | | | | | | | | | | |
|---|---|---|---|---|---|---|---|---|---|---|
| Note 1) A value measured by SEM observation. Note 2) A value measured by a laser diffraction scattering type particle diameter distribution measuring device. Note 3) "-" indicates not measured. | | | | | | | | | | |

**[Table 4-2]**

| Table 4-2 Properties of alumina powder | | | | | | | | |
|---|---|---|---|---|---|---|---|---|
| | | Example 10 | Example 11 | Example 12 | Comparative Example 1 | Comparative Example 2 | Comparative Example 3 | Comparative Example 4 |
| Average particle diameter^{Note 1} (µm) | | 66 | 106 | 89 | - | - | - | 12 |
| Average particle diameter^{Note 2} (µm) | | 57 | - | 77 | 18 | 50 | 95 | - |
| Average thickness^{Note 2} (µ m) | | 1.2 | 0.85 | 0.64 | - | - | - | 0.38 |
| Average aspect ratio | | 55 | 125 | 139 | 1.5 | 1.2 | 1.1 | 32 |
| Crystalline phase | | Corundum | Corundum | Corundum | Corundum | Corundum | Corundum | Corundum |
| Na₂O (mass%) | | 0.4 | 1.6 | 0.6 | 0.07 | 0.32 | 0.37 | 0.38 |
| SiO₂ (mass%) | | 0.6 | 1.2 | 0.6 | 0.06 | 0.01 | 0.03 | 0.05 |
| Fe₂O₃ (mass%) | | 0.02 | 0.02 | 0.02 | 0.02 | 0.02 | 0.02 | 0.02 |
| F (mass%) | | - | - | - | - | - | - | 0.3 |
| LOI (mass%) | | - | - | - | 0.02 | 0.02 | 0.01 | 0.03 |
| S_{BET} (m²/g) | | 0.4 | 0.7 | 0.5 | 0.7 | 1. 0 | 0.8 | 0.6 |
| Luminosity intensity Si | 15° | - | - | - | 0.00 | 0.00 | 0.00 | 0.00 |
| | 45° | - | - | - | 0.00 | 0.00 | 0.00 | 0.00 |
| | 75° | - | - | - | 0.00 | 0.00 | 0.00 | 0.00 |
| Luminosity area Sa | 15° | - | - | - | 0.00 | 0.00 | 0.00 | 0.00 |
| | 45° | - | - | - | 0.00 | 0.00 | 0.00 | 0.00 |
| | 75° | - | - | - | 0.00 | 0.00 | 0.00 | 0.00 |

| | | | | | | | | |
|---|---|---|---|---|---|---|---|---|
| Note 1) A value measured by SEM observation. Note 2) A value measured by a laser diffraction scattering type particle diameter distribution measuring device. Note 3) "-" indicates not measured. | | | | | | | | |

## Claims

1. A high-luminance thin-layered α-alumina powder having an average particle diameter of 20 µm or more and 200 µm or less, an average thickness of 0.20 µm or more and 2.00 µm or less, and an average aspect ratio of 50 or more and 600 or less, and
having a luminosity intensity Si value at measurement angles θ of 15° and 45° in a range of 1.00 or more and 15.00 or less in a luminosity measurement.

2. The high-luminance thin-layered α-alumina powder according to claim 1, wherein the average particle diameter is 20 µm or more and 120 µm or less, and the average thickness is 0.20 µm or more and 0.50 µm or less.

3. The high-luminance thin-layered α-alumina powder according to claim 1, wherein an alkali metal (AM) is contained in an amount of 0.2 mass% or more and 5.0 mass% or less in terms of AM₂O, and silicon (Si) is contained in an amount of 0.3 mass% or more and 10.0 mass% or less in terms of SiO₂.

4. The high-luminance thin-layered α-alumina powder according to claim 3, wherein the alkali metal (AM) is sodium (Na) and/or potassium (K).

5. The high-luminance thin-layered α-alumina powder according to any one of claims 1 to 4, wherein the average particle diameter is 50 µm or more and 120 µm or less.

6. The high-luminance thin-layered α-alumina powder according to any one of claims 1 to 4, wherein the luminosity intensity Si value is in a range of 5.00 or more and 15.00 or less.

7. A method for producing the high-luminance thin-layered α-alumina powder according to any one of claims 1 to 4, comprising:
a step of adding an additive to a thin-layered boehmite powder and then performing mixing to prepare a raw material mixture; and
a step of calcining the raw material mixture at a temperature in a range of 1,000°C or higher and 1,300°C or lower,
the raw material mixture containing 0.5 mass% or more and 10.0 mass% or less of an alkali metal (AM) in terms of AM₂O, 0.1 mass% or more and 10.0 mass% or less of silicon (Si) in terms of SiO₂, and 0.1 mass% or more and 5.0 mass% or less of fluorine (F).

8. The method according to claim 7, wherein the thin-layered boehmite powder has an average particle diameter of 2 µm or more and 15 µm or less and an average aspect ratio of 20 or more and 60 or less.

9. The method according to claim 7, wherein the additive contains aluminum fluoride (AlF₃) and silicon oxide (SiO₂).

10. The method according to claim 9, wherein the additive further contains an oxide (AM₂O) and/or a carbonate (AM₂CO₃) of the alkali metal (AM).

11. The method according to claim 7, wherein the additive contains an alkali fluorosilicate (AM₂SiF₆).

12. The method according to claim 7, wherein the alkali metal (AM) is sodium (Na) and/or potassium (K).

13. A coating or cosmetic comprising the high-luminance thin-layered α-alumina powder according to any one of claims 1 to 4.
